# EUROPÄISCHE PATENTANMELDUNG

(11) **EP 3 597 632 A1**
(43) Veröffentlichungstag der Anmeldung: **22.01.2020**
(21) Anmeldenummer: 18184144.6
(22) Anmeldetag: 18.07.2018
(51) Int. Cl.: C07C 263/20, B01D 1/06, B01D 1/22, C08G 18/32, C08K 5/29, C08K 13/08

(54) **VERFAHREN ZUR RÜCKGEWINNUNG VON DIISOCYANATEN AUS DESTILLATIONSRÜCKSTÄNDEN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Verfahren zur Abtrennung eines bei Raumtemperatur festen Diisocyanats aus einem Destillationsrückstand, aus einem Herstellungsprozess des Diisocyanats, umfassend die folgenden Schritte:
(i) Vermischen des Destillationsrückstandes mit einem Bitumen in der Weise, dass eine Mischung erhalten wird, welche 70 bis 90 Gew.-% des Destillationsrückstandes und 10 bis 30 Gew.-% des Bitumens enthält, jeweils bezogen auf die Mischung,
(ii) Destillation der Mischung in einem Dünnschichtverdampfer oder einem Fallfilmverdampfer, wobei ein Sumpfablauf und ein gasförmiger Produktstrom erhalten wird,
(iii) Kondensation des gasförmigen Produktstroms und Erhalt eines Feststoffs enthaltend das bei Raumtemperatur feste Diisocyanat.

Außerdem sind die Verwendung eines Dünnschichtverdampfers oder Fallfilmverdampfers, eine das bei Raumtemperatur feste Diisocyanat enthaltende Zusammensetzung und ein Verfahren zur Herstellung eines Elastomers aus dieser Zusammensetzung sowie das Elastomer selbst weitere Gegenstände der Erfindung.

## Beschreibung

Die vorliegende Erfindung betrifft ein Verfahren zur Abtrennung eines bei Raumtemperatur festen Diisocyanats aus einem Destillationsrückstand, der bei einem Herstellungsprozess des Diisocyanats entsteht. Ein weiterer Gegenstand der Erfindung ist das nach diesem Verfahren erhältliche bei Raumtemperatur feste Diisocyanat. Außerdem sind die Verwendung eines Dünnschichtverdampfers oder Fallfilmverdampfers, eine das bei Raumtemperatur feste Diisocyanat enthaltende Zusammensetzung und ein Verfahren zur Herstellung eines Elastomers aus dieser Zusammensetzung sowie das Elastomer selbst weitere Gegenstände der Erfindung.

Die großtechnische Herstellung von Diisocyanaten durch Umsetzung von Aminen mit Phosgen in Lösungsmitteln ist bekannt und in der Literatur ausführlich beschrieben (Ullmanns Encyklopädie der technischen Chemie, 4. Auflage, Band 13, Seite 347-357, Verlag Chemie, GmbH, D-6940 Weinheim, 1977 oder auch EP 1 575 908 A1). Bei der Aufreinigung der mit diesen Verfahren hergestellten Diisocyanate durch Destillationsprozesse fällt ein Destillationsrückstand an. Dieser Rückstand enthält, abhängig von der chemischen Natur des hergestellten Diisocyanats, einen beträchtlichen Anteil an monomerem Diisocyanat. Der Rückstand kann durch Verbrennen entsorgt werden, wobei jedoch auch das darin enthaltene monomere Diisocyanat verloren geht.

Daher sind verschiedene Verfahren entwickelt worden, um das im Rückstand enthaltene monomere Diisocyanat von dem Destillationsrückstand abzutrennen und zurückzugewinnen. Dazu werden die Rückstände mit hochsiedenden Kohlenwasserstoffen wie beispielsweise Bitumen vermischt und dann in einem Knetertrockner, Schaufeltrockner oder Batch-Destillationsverfahren das im Rückstand enthaltene Diisocyanat abgetrennt.

Wenn der Rückstand eine hohe Viskosität bis hin zu einem festen Zustand aufweist und die monomeren Diisocynate bei Raumtemperatur nicht flüssig sind, muss vor einer Rückgewinnung die Viskosität des Rückstands erniedrigt werden, um überhaupt ein Abtrennungsverfahren durchführen zu können. Ein Erhitzen nicht flüssiger Rückstände erniedrigt zwar ihre Viskosität, es kommt aber auch zu unerwünschten Nebenreaktionen - z.B. Oligomerisierungen - des zu isolierenden Diisocyanates. Eine weitere Herausforderung bei der Rückgewinnung ist, dass bei destillativen Verfahren der Sumpfablauf, d.h. der Teil der von dem abgetrennten Diisocyanat verbleibt, auch eine hohe Viskosität aufweisen kann und daher nur schwer aus der Trennapparatur entfernt werden kann. Diese hohe Viskostität beruht bei herkömmlichen Rückgewinnungsverfahren häufig auf Oligomerisierungen von noch in dem Sumpfablauf enthaltenen Diisocyanat, die durch hohe Temperaturen hervorgerufen werden.

Naphthalindiisocyanat (NDI) hat eine breite industrielle Anwendung gefunden und kann mit bekannten, eingangs beschrieben Verfahren wie z.B. Phosgenierung, aus Naphthalindiamin (NDA) hergestellt werden. Da es sich bei monomerem Naphtalindiisocyanat um einen Feststoff handelt, stellt die Aufreinigung des Produkts verglichen mit anderen, bei Verarbeitungstemperatur flüssigen Isocyanaten, wie beispielsweise Toluylendiisocyanat (TDI), grundsätzlich eine Herausforderung dar. Die Schwierigkeiten werden durch die physikalischen Eigenschaften von Naphtalindiisocyanat noch vergrößert, weil es bei 127 °C schmilzt und bereits bei 130 °C zu sublimieren beginnt. Der Rückstand aus dem Aufreinigungsprozess von Naphtalindiisocyanat enthält noch ca. 70-80 Gew.-% monomeres Naphtalindiisocyanat. Jährlich werden von 150 bis 300 Tonnen an Naphtalindiisocyanat verbrannt, die im Rückstand des Aufreinigungsverfahrens enthalten sind. Bei der Rückgewinnung von monomeren Naphtalindiisocyanat in einem Batch-Destillationsverfahren, werden ca. 50 % des in dem Rückstand enthaltenen monomeren Napthalindiisocyanats zurückgewonnen. Üblicherweise wird der Rückstand bei diesem Verfahren in ca. 30 Gew.-% Bitumen gelöst.

Die EP 0 626 368 A1 beschreibt ein Verfahren zur Herstellung von reinen, destillierten Isocyanaten bei dem der Rückstand mit 2 bis 50 Gew.-% hochsiedenden Kohlenwasserstoffen versetzt wird und das Isocyanat aus diesem Rückstand bei Temperaturen von 160 °C bis 280 °C extrahiert wird. In einem Ausführungsbeispiel wird Rückstand welcher das bei Raumtemperatur flüssige Toluylendiisocyanat enthält, mit 12 Gew.-% Bitumen B80, das nach neuer Kennzeichnung Bitumen 70/100 entspricht, versetzt und in einen auf 240 °C erhitzten Knetertrockner gegeben. Bei diesem Verfahren werden Knetertrockner eingesetzt, die eine relativ aufwendige Technik mit mechanisch bewegten Teilen bedeuten. Außerdem muss bei diesem Verfahren gewährleistet werden, dass kontinuierlich rieselfähiges Material aus dem Knetertrockner ausgetragen wird, so dass die Anwendung des Verfahrens nicht für alle Arten von Rückständen geeignet ist. Die Druckschrift bezieht sich zwar auch auf Naphtalindiisocyanat, offenbart im experimentellen Teil jedoch nur Toluylendiisocyanat. Auf die besonderen Schwierigkeiten der Rückgewinnung eines bei Raumtemperatur festen Diisocyanats wird nicht eingegangen.

EP 2 872 481 offenbart ein Sprühtrocknungsverfahren für die Aufreinigung von Destillationsrückständen, die bei der Herstellung von Isocyanat anfallen. Dabei wird der Rückstand zusammen mit einem Trägergas wie Stickstoff, Edelgasen oder Kohlenstoffdioxid, in einen Reaktor eingesprüht und dass monomere Diisocyanat abgetrennt. Dieses Verfahren ist jedoch nur durchführbar, wenn die Temperatur des Destillationsrücksstand so hoch ist, dass die Viskosität des Rückstands ausreichend klein ist um eingesprüht zu werden. Die Druckschrift offenbart Temperaturen des Rückstands von 20 bis 300 °C. Das einzige Ausführungsbeispiel bezieht sich auf das bei Raumtemperatur flüssige Toluylendiisocyanat. Auf die besonderen Schwierigkeiten der Rückgewinnung eines bei Raumtemperatur festen Diisocyanats wird nicht eingegangen.

Die US 3,694,323 offenbart ein Verfahren zur Rückgewinnung eines Isocyanates aus einem Rückstand unter Zuhilfenahme eines weiteren Isocyanats, welches einen höheren Siedepunkt als das zu reinigende Isocyanat aufweist und somit die Viskosität des Rückstands erniedrigt und eine Reinigung ermöglicht. Nachteilig an diesem Verfahren ist jedoch, dass das das Verfahren einen verhältnismäßig hohen Energiebedarf hat, da in den Beispielen für die Reinigung von TDI Temperaturen zwischen 190 °C und 250 °C benötigt werden. Auch die Druckschrift bezieht sich allgemein auf Naphtalindiisocyanat, offenbart im experimentellen Teil jedoch nur Toluylendiisocyanat und enthält keine Lehre zu den besonderen Schwierigkeiten bei der Rückgewinnung eines bei Raumtemperatur festen Diisocyanats.

WO 2007/036479 A1 bezieht sich auch auf Destillationsrückstände, die nicht flüssig sind und offenbart ein Verfahren zur Rückgewinnung eines Isocyanats, bei dem der Rückstand während der gesamten Verweilzeit im Apparat zur Rückgewinnung bei einer Temperatur von 210 bis 330 °C und einem Druck unter 300 hPa eine hochviskose Flüssigkeit und/oder einen nicht-versprödenden Feststoff bildet und durch eine Zwangsförderung für nicht-feste Medien aus diesem Apparat ausgetragen wird. Die Druckschrift bezieht sich nicht darauf, dass in dem Hilfs- oder Zusatzstoffe verwendet werden.

DE 2035773 offenbart ein Rückgewinnungsverfahren, bei dem das Diisocyanat aus dem Rückstand mit Hilfe eines Fallrohrverdampfers zurückgewonnen wird. Die Temperatur, bei der das Verfahren durchgeführt wird beträgt bis zu 250 °C. Im experimentellen Teil wird eine Durchführungstemperatur von 189 °C und ein Druck von 9 hPa offenbart.

Die Anmeldung EP 17151971.3 EP beschreibt ein Verfahren zur Rückgewinnung eines Isocyanates aus seinem Phosgenierungsrückstand unter Verwendung von ≥ 32 Gew.-% Bitumen als Lösemittel.

Nachteilig an den herkömmlichen Verfahren zur Rückgewinnung von Diisocyanat aus dem Destillationsrückstand ist die Verwendung von größeren Mengen eines Zusatzmittels welches das zurückgewonnene Diisocyanat verunreinigen kann. Darüber hinaus sind Verfahren bei denen Knetertrockner oder Schaufeltrockner verwendet werden, durch einen hohen Investitions- und Instandhaltungsaufwand gekennzeichnet. Des Weiteren erfordert die Durchführung der meisten herkömmlichen Verfahren bei hohen Temperaturen von mehr als 180 °C viel Energie. Zudem führen diese Temperaturen zu unerwünschten Oligomerisierungsreaktionen des monomeren Diisocyanats. Nachteilig an den bekannten Batch-Destillationsverfahren ist, dass es sich nicht um kontinuierliche Prozesse handelt.

Es besteht daher ein Bedarf für ein Verfahren für die Rückgewinnung von Diisocyanaten, insbesondere von bei Raumtemperatur festen Diisocyanaten wie Naphtalindiisocyanat, aus Destillationsrückständen die bei der Herstellung von Diisocyanaten anfallen, bei dem ein zumindest der gleiche oder ein höherer Anteil des im Rückstand enthaltenen Diisocyanats zurückgewonnen werden kann als bei herkömmlichen Verfahren und das gleichzeitig weniger Energie und Zusatzsstoffe verbraucht. Vorteihaft sind zudem Verfahren, die als kontinuierlicher Prozess durchgeführt werden können. Außerdem sollte das zurückgewonnene Diisocyanat möglichst frei von Fremdstoffen, wie beispielsweise Zusatzsstoffen aus dem Rückgewinnungsprozess sein.

Daher war es Aufgabe der vorliegenden Erfindung ein Verfahren zur Rückgewinnung von bei Raumtemperatur festen Diisocyanaten, insbesondere von Naphthalindiisocyanat, aus einem Destillationsrückstand bereitzustellen, mit dem mindestens ein gleicher oder sogar ein höherer Anteil des im Rückstand enthaltenen Diisocyanats, insbesondere von Naphtalindiisocyanat, zurückgewonnen werden kann, als mit herkömmlichen Verfahren. Gleichzeitig soll das erfindungsgemäße Verfahren weniger Energie und weniger Zusatzstoffe verbrauchen als herkömmliche Verfahren. Des Weiteren soll das zurückgewonnene Diisocyanat möglichst frei von Fremdstoffen, wie beispielsweise Zusatzsstoffen aus dem Rückgewinnungsprozess sein, so dass es ohne weitere Reinigungsprozesse als Edukt für weitere Synthesen, z.B. Polymersynthesen verwendet werden kann.

Diese Aufgabe wurde gelöst durch ein Verfahren zur Abtrennung eines bei Raumtemperatur festen Diisocyanats aus einem Destillationsrückstand, aus einem Herstellungsprozess des Diisocyanats, umfassend die folgenden Schritte:
(i) Vermischen des Destillationsrückstandes mit einem Bitumen in der Weise, dass eine Mischung erhalten wird, welche 70 bis 90 Gew.-% des Destillationsrückstandes und 10 bis 30 Gew.-% des Bitumens enthält, jeweils bezogen auf die Mischung,
(ii) Destillation der Mischung in einem Dünnschichtverdampfer oder einem Fallfilmverdampfer, wobei ein Sumpfablauf und ein gasförmiger Produktstrom erhalten wird,
(iii) Kondensation des gasförmigen Produktstroms und Erhalt eines Feststoffs enthaltend das bei Raumtemperatur feste Diisocyanat.

Überraschend wurde nun gefunden, dass auch bei Raumtemperatur feste Diisocyanate aus einem Destillationsrückstand mit vergleichsweise geringen Mengen eines Bitumens in einem Dünnschichtverdampfer und/oder Fallfilmverdampfer zurückgewonnen werden können, wobei mindestens ein gleicher oder sogar ein höherer Anteil an Diisocanat zurückgewonnen wird als bei vergleichbaren Verfahren. Trotz eines im Vergleich zu herkömmlichen Verfahren geringeren Anteil an Bitumens ist es zudem möglich die Mischung des Rückstandes mit dem Bitumen über den gesamten Destillationsprozess hinweg flüssig zu halten. Des Weiteren weist das zurückgewonnene Diisocyanat einen sehr geringen Grad an Verunreinigungen auf.

Vorliegend wird unter "bei Raumtemperatur festen Diisocyanaten" verstanden, dass die Diisocyanate bei 23 °C und Normaldruck im festen Aggregatzustand vorliegen.

Vorliegend wird Naphthalindiisocyanat als Oberbegriff für die möglichen Isomere oder deren Gemische verstanden. Beispiele für solche Isomere sind 1,5-Naphthalindiisocyanat oder 1,8-Naphthalindiisocyanat.

Geeignete Dünnschichtverdampfer sind beispielsweise Starrflügelrotor-Dünnschichtverdampfer oder Wischerklappen-Dünnschichtverdampfer oder Kurzwegverdampfer.

Geeignete Fallfilmverdampfer sind beispielsweise Rohrbündel-Fallrohrverdampfer oder Wendelrohrverdampfer.

Bitumen, insbesondere der unterschiedliche Schwefelgehalt verschiedener Bitumen und die Herstellung von Bitumen sind dem Fachmann bekannt. Bevorzugt weist das Bitumen der Mischung aus Schritt (i) einen Schwefelgehalt von 2,5 bis 3,1 Gew.-% auf, bevorzugter von 2,7 bis 3,1 Gew.-%, besonders bevorzugt von 2,9 bis 3,08 Gew.-%, jeweils bezogen auf das Bitumen. In einer bevorzugten Ausführungsform ist das Bitumen der Mischung aus Schritt (i) Bitumen 160/220 der Firma Shell.

Bevorzugt enthält der Destillationsrückstand in Schritt (i) von 30 bis 70 Gew.-% des bei Raumtemperatur festen Diisocyanats, bevorzugter 40 bis 60 Gew.-%, besonders bevorzugt 45 bis 50 Gew.-%, jeweils bezogen auf den Destillationsrückstand.

Bevorzugt werden in Schritt (i) 85 bis 70 Gew.-% des Destillationsrückstands mit von 15 bis 30 Gew.-% des Bitumens vermischt, jeweils bezogen auf die Summe der Massen des Destillationsrückstands und des Bitumens. Hieraus ergibt sich der zusätzliche Vorteil, dass die Ausbeute an rückgewonnenem bei Raumtemperatur festem Diisocyanat weiter gesteigert werden kann und die Mischung über den gesamten Destillationsprozess hinweg flüssig gehalten wird.

In einer weiteren bevorzugten Ausführungsform ist das bei Raumtemperatur feste Diisocyanat 1,5-Naphthalindiisocyanat, 1,8-Naphthalindiisocyanat, 1,4-Phenylendiisocyanat, Tetralindiisocyanat, o-Tolidindiisocyanat, Duroldiisocyanat, Benzidindiisocyanat und/oder 1,4-Anthrylendiisocyanat, bevorzugter 1,5-Naphthalindiisocyanat, 1,8-Naphthalindiisocyanat, 1,4-Phenylendiisocyanat, Tetralindiisocyanat und/oder o-Tolidindiisocyanat und besonders bevorzugt 1,5-Naphthalindiisocyanat und/oder 1,8-Naphthalindiisocyanat.

Prinzipiell kann die Herstellung der bei Raumtemperatur festen Diisocyanate auf beliebigen Wegen, beispielsweise durch Umsetzung der entsprechenden Diamine oder deren Salze mit Phosgen, erfolgen. Wichtig ist jeweils nur, dass bei dem genutzten Verfahren mindestens ein, bei Raumtemperatur feste Diisocyanate enthaltender Destillationsrückstand übrig bleibt, der dann im erfindungsgemäßen Schritt (i) eingesetzt werden kann.

Besonders bevorzugt ist es, dass der Herstellungsprozess des Diisocyanats eine Phosgenierung eines Diamine, bevorzugter eine Flüssigphasen-Phosgenierung eines Diamins.

Bevorzugt wird in dem Herstellungsprozess des Diisocyanats ein Diamin verwendet, dass bis auf die Isocyanatgruppen die Struktur des Diisocyanats aufweist, bei dem jedoch im Herstellungsprozess die beiden Aminogruppen gegen Isocyanatgruppen ausgetauscht werden. Beispielhaft sei 1,5-Naphthalindiamin als entsprechendes Diamin zum 1,5-Naphthalindiisocyanat genannt. Wenn das bei Raumtemperatur feste Diisocyanat ein Isomerengemisch ist, kommt eine entsprechende Isomerenmischung an Diaminen zum Einsatz.

Die kontinuierliche Herstellung von organischen Isocyanaten durch Reaktion von primären organischen Aminen mit Phosgen ist vielfach beschrieben und wird im großtechnischen Maßstab durchgeführt (siehe z. B. Ullmanns Encyclopedia of Industrial Chemistry, Wiley-VCH Verlag GmbH & Co KGaA, Weinheim, Online ISBN: 9783527306732, DOI: 10.1002/14356007.a14_611, S. 63 ff (2012)). Besonders bevorzugt wird das bei Raumtemperatur feste Diisocyanat aus dem entsprechenden Diamin unter Verwendung von Phosgen nach dem aus der WO 2014/044699 A1 bekannten Verfahren hergestellt, welches die folgenden Schritte umfasst:
(A) Herstellung einer Suspension des entsprechenden Diamins in einem inerten Lösungsmittel, wobei das Diamin mittels eines dynamischen Mischaggregats in dem Lösungsmittel verteilt wird,
(B) Phosgenierung des in dem inerten Lösungsmittel suspendierten Diamins unter Erhalt des jeweiligen Diisocyanats,
wobei das dynamische Mischaggregat in Schritt (A) ausgewählt ist aus der Gruppe bestehend aus Dispergierscheiben und Rotor-Stator-Systemen, bevorzugt Rotor-Stator-Systemen, besonders bevorzugt Kolloidmühlen, Zahndispergiermaschinen und Drei-Walzenstühlen. Ganz besonders bevorzugt sind Zahndispergiermaschinen als dynamische Mischaggregate.

Die im vorstehenden Absatz genannten Dispergierscheiben und Rotor-Stator-Systeme haben die gleiche Bedeutung wie auf Seite 4, Zeile 17 bis Seite 5, Zeile 11 der WO 2014/044699 A1 5 offenbart.

Geeignete inerte Lösungsmittel sind aromatische Lösungsmittel, die auch halogeniert sein können. Beispiele hierfür sind Toluol, Monochlorbenzol, o-, m- oder p-Dichlorbenzol, Trichlorbenzol, Chlortoluole, Chlorxylole, Chlorethylbenzol, Chlornaphthaline, Chlordiphenyle, Xylole, Dekahydronaphthalin, Benzol oder Gemische der vorstehenden Lösungsmittel. Weitere Beispiele 10 für geeignete organische Lösungsmittel sind Methylenchlorid, Perchlorethylen, Hexan, Diethylisophthalat, Tetrahydrofuran (THF), Dioxan, Trichlorfluormethan, Butylacetat und Dimethylformamid (DMF). Bevorzugt wird Monochlorbenzol oder o-Dichlorbenzol oder eine Mischung aus beiden eingesetzt; besonders bevorzugt wird Monochlorbenzol eingesetzt.

Bei der Reaktion in Schritt (B) wird Phosgen im Überschuss eingesetzt. Das heißt, pro mol Amingruppen wird mehr als ein mol Phosgen eingesetzt. Das molare Verhältnis Phosgen zu Amingruppen beträgt demnach von 1,01 : 1 bis 20 : 1, bevorzugt 1,1 : 1 bis 10 : 1, besonders bevorzugt 1,1 : 1 bis 5,0 : 1. Ggf. kann dem Reaktionsgemisch während der Umsetzung weiteres Phosgen oder Phosgenlösung zugeführt werden, um einen ausreichenden Phosgenüberschuss aufrecht zu erhalten bzw. um einen Verlust an Phosgen wieder auszugleichen.

Die Reaktion kann kontinuierlich und diskontinuierlich durchgeführt werden. Als Reaktoren kommen Rührkessel, Rohrreaktoren, Sprühtürme oder auch Schlaufenreaktoren in Betracht. Prinzipiell lassen sich aber auch andere Bauformen, die hier nicht exemplarisch aufgeführt wurden, nutzen. Bevorzugt wird diskontinuierlich gearbeitet.

Die Reaktion kann bis zum vollständigen Umsatz zum Isocyanat innerhalb der ersten Reaktionsstufe geführt werden. Es kann aber auch vorteilhaft oder notwendig sein, einen Teilumsatz, insbesondere von Resten Aminhydrochlorid, in einem Nachreaktor durchzuführen. Bei dem Nachreaktor kann es sich um übliche Reaktorbauformen unterschiedlichen Rückvermischungsgrades, wie Rührkesseln, Schlaufenreaktoren oder Rohrreaktoren handeln. Es kann weiterhin vorteilhaft sein, das Reaktionsgemisch gemäß seiner Partikelgrößenverteilung in Teilströme aufzuteilen, und getrennt einem oder mehreren Nachreaktoren zuzuführen. Als Bauformen für die Abtrennung kommen bekannte Apparate wie beispielsweise Filter, Zyklone oder Schwerkraftabscheider in Frage. Die Teilströme können dabei vor oder während der Umsetzung mit entsprechenden mechanischen Verfahren zur Einstellung der Partikelgröße behandelt werden, z. B. durch Mahlen.

Das nicht umgesetzte Phosgen wird zumeist, gegebenenfalls nach einer Reinigung, zurückgeführt und wieder zur Phosgenierung eingesetzt.

Um das Diisocyanat von dem Lösemittel zu trennen bieten sich die dem Fachmann bekannten Methoden wie beispielsweise Kristallisation, Sublimation oder Destillation gegebenenfalls unter Zugabe von beispielsweise Impfkristallen oder Schleppmitteln. Bevorzugt wird ein Verfahren mit Kristallisation oder Destillation eingesetzt. Bei den oben genannten Methoden bleibt ein hochviskoser oder sogar fester Rückstand, der einen variierend hohen Anteil an Diisocyanaten enthält. Bevorzugt können Rückstände aus den Methoden Kristallisation ud Sublimation als Destillationsrückstand für das erfindungsgemäße Verfahren eingesetzt werden.

In einer weiteren bevorzugten Ausführungsform enthält der im erfindungsgemäßen Verfahren eingesetzte Rückstand ≥ 0 Gew.-% bis ≤ 4 Gew.-%, bevorzugt ≥ 0,001 Gew.-% bis ≤ 2 Gew.-% und besonders bevorzugt ≥ 0,01 Gew.-% bis ≤ 1 Gew.-%, jeweils bezogen auf die Gesamtmenge des bei Raumtemperatur feste Diisocyanate enthaltenden Rückstands, an monomeren Diisocyanaten, deren Siedetemperatur oberhalb der Siedetemperatur des bei Raumtemperatur festen Diisocyanats liegt und die sich von diesen unterscheiden. Dies ist besonders vorteilhaft, da auch ohne diesen Zusatz eine hohe Ausbeute bei der Rückgewinnung erzielt werden kann und das gereinigte bei Raumtemperatur feste Diisocyanat somit weitestgehend frei von diesen Verunreinigungen bleibt.

Die Bestimmung der Gew.-% an monomeren Diisocyanaten, deren Siedetemperatur oberhalb der Siedetemperatur des bei Raumtemperatur festen Diisocyanats liegt, erfolgt gaschromatographisch mittels FID-Detektor, vorzugsweise unter Verwendung einer Optima 5 Säule und folgenden Parametern: Split-Rate: 8.31:1 mL/min; Flussrate: 96,4 mL/min Druck: 0,7 bar, Trägergas: Helium, Injektionsvolumen: 1 *µL*, Inliner: gerader Split-Liner gefüllt mit Carbofritt, wobei bei der Auswertung die Flächenprozent gleich der Gewichtsprozent gesetzt werden.

In einer weiteren bevorzugten Ausführungsform weist der Rückstand bei der Behandlung in Schritt (ii) eine mittlere Verweilzeit von ≥ 1 bis ≤ 15 Minuten, bevorzugt von ≥ 1 bis ≤ 10 Minuten und besonders bevorzugt von ≥ 1 bis ≤ 5 Minuten in dem mindestens einen Dünnschichtverdampfer und/oder Fallfilmverdampfer auf, besonders bevorzugt weist der Rückstand diese Verweilzeit in einem handelsüblichen Fallfilmverdampfer aus Glas, mit einer Verdampferfläche von 0.1 m² (Durchmesser 100 mm, Länge 300 mm) auf. Hieraus ergibt sich der Vorteil, dass die Wahrscheinlichkeit von unerwünschten Nebenreaktionen - wie beispielsweise Oligomerisierungen - weiter reduziert werden kann.

Bevorzugt wird Schritt (ii) des Verfahrens bei einer Temperatur von 130 °C bis 160 °C und einem Druck von 0,4 bis 4 mbar durchgeführt, bevorzugter von 140°C bis 160 °C und von 0,6 mbar bis 2 mbar, besonders bevorzugt von 150 °C bis 160 °C und von 0,7 bis 1,5 mbar. Hieraus ergibt sich der Vorteil, dass die Entstehung von Nebenprodukten, bzw. die Oligomerisierung des Diisocyanats während der Destillation weitestgehend unterdrückt werden kann.

Bevorzugt ist der Sumpfablauf aus Schritt (ii) bei der vorherrschenden Temperatur am Ausgang des Dünnschichtverdampfers oder Fallfilmverdampfers kein Feststoff. Bevorzugt wird der Sumpfablauf kontinuierlich aus der Destillationsappartur ausgeschleust und danach entweder recycliert, z.B. durch Verbrennung zur Wärmegewinnung, oder verworfen. Besonders bevorzugt ist der Sumpfablauf unter den Destillationsbedinungen flüssig.

In einer weiteren bevorzugten Ausführungsform wird bei der Destillation in Schritt (iii) ein Kühlmittel verwendet, wobei die Kühlmitteltemperatur bevorzugt unterhalb des Schmelzpunktes des bei Raumtemperatur festen Diisocyanats liegt. Das Kühlmittel dient der schnellen Kondensation des Produktstromes.

Bevorzugt enthält der Feststoff aus Schritt (iii) wenigstens 95 Gew.-% des bei Raumtemperatur festen Diisocyanats, bevorzugter wenigstens 97 Gew.-%, besonders bevorzugt wenigstens 99 Gew.%, jeweils bezogen auf den Feststoff. Bevorzugt wird der Anteil an festem Diisocyanat im Feststoff durch gaschromatographische Methoden bestimmt. Durch den reduzierten Einsatz von Hilfsstoffen wie Bitumen in Schritt (i) des Verfahrens wird die Kontamination des aus dem Rückstand gewonnenen, monomerem bei Raumtempratur festen Diisocyanats minimiert.

Bevorzugt werden durch das Verfahren ≥ 60 Gew.-%, bevorzugt ≥ 70 Gew.-% und ganz besonders bevorzugt ≥ 80 Gew.-%, der noch im Rückstand in Schritt (i) enthaltenen bei Raumtemperatur festen Diisocyanate zurückgewonnen.

Eine weitere Ausführungsform der Erfindung betrifft die Verwendung einer Mischung enthaltend 70 bis 90 Gew.-% eines Destillationsrückstandes aus einem Herstellungsprozess eines bei Raumtemperatur festen Diisocyanats und 10 bis 30 Gew.-% eines Bitumens, jeweils bezogen auf die Mischung, in einem Verfahren zur Abtrennung des Diisocyanats durch Destillation mittels eines Dünnschichtverdampfers oder Fallfilmverdampfers.

Bevorzugt kann der Feststoff enthaltend das bei Raumtemperatur feste Diisocyanat aus Schritt (iii) des Verfahrens alleine oder auch in Mischungen mit einem direkt aus der ersten Reinigungsstufe nach der Phosgenierungsreaktion erhaltenen Diisocyanat allen dem Fachmann geläufigen Verwendungszwecken zugeführt werden. In einer Ausführungsform wer zur Herstellung von Hochleistungs-Elastomeren, wie beispielsweise Vulkollan®, verwendet werden. Insbesondere bevorzugt ist die Weiterverarbeitung des Feststoffs mit NCO-reaktiven Verbindungen wie Polyolen zu Polyurethanen, ggf. über Präpolymere als Zwischenstufen, zu nennen.

Eine weitere Ausführungsform der Erfindung betrifft eine Zusammensetzung umfassend einen Feststoff enthaltend ein bei Raumtemperatur festes Diisocyanat aus Schritt (iii) eines Verfahrens nach einem der Ansprüche 1 bis 9 und mindestens eine NCO-reaktive Verbindung, bevorzugt mindestens ein Polyesterpolyol.

Weitere Gegenstände der vorliegenden Erfindung sind ein Verfahren zur Herstellung eines Elastomers, bei dem mindestens eine erfindungsgemäße Zusammensetzung gegebenenfalls unter Erwärmen chemisch umgesetzt wird und das nach diesem Verfahren hergestellte oder herstellbare Elastomer.

Bevorzugt sind diese Elastomere Polyurethanen, die ggf. über Präpolymere als Zwischenstufen, erhalten werden.

Diese Polyurethane haben bevorzugt Rohdichten von 200 kg/m³ bis 1400 kg/m³, besonders bevorzugt von 600 kg/m³ bis 1400 kg/m³ und ganz besonders bevorzugt von 800 kg/m³ bis 1400 kg/m³. Ganz besonders bevorzugt werden zellige oder massive Gießelastomere hergestellt, ganz besonders bevorzugt Gießelastomere auf Polyesterpolyol Basis.

Die oben beschriebende Zusammensetzung kann bevorzugt übliche Hilfs- und Zusatzmittel, wie z.B. Rheologieverbesserer (zum Beispiel Ethylencarbonat, Propylencarbonat, dibasische Ester, 10 Zitronensäureester), Stabilisatoren (zum Beispiel Broenstedt- und Lewis-Säuren, wie etwa Salzsäure, Phosphorsäure, Benzoylchlorid, Organomineralsäuren wie Dibutylphosphat, weiterhin Adipinsäure, Äpfelsäure, Bernsteinsäure, Traubensäure oder Zitronensäure), UV-Schutzmittel (zum Beispiel 2,6-Dibutyl-4-methylphenol), Hydrolyseschutzmittel (zum Beispiel sterisch gehinderte Carbodiimide), Emulgatoren sowie Katalysatoren (zum Beispiel Trialkylamine, 15 Diazabicyclooctan, Zinndioctoat, Dibutylzinndilaurat, N-Alkylmorpholin, Blei-, Zink-, Zinn-, Kalzium-, Magnesiumoctoat, die entsprechenden Naphthenate und p-Nitrophenolat und/oder auch Quecksilberphenylneodecanoat) und Füllstoffe (zum Beispiel Kreide), gegebenenfalls in das/den später zu bildende/n Polyurethan/Polyharnstoff einbaufähige Farbstoffe (die also über Zerewitinoff-aktive Wasserstoffatome verfügen) und/oder Farbpigmente enthalten.

Als NCO-reaktive Verbindungen können alle dem Fachmann bekannten Verbindungen eingesetzt werden.

Als NCO-reaktive Verbindungen werden bevorzugt Polyetherpolyole, Polyesterpolyole, Polycarbonatpolyole und Polyetheramine, welche eine mittlere OH- bzw. NH-Funktionalität von mindestens 1,5 aufweisen, sowie kurzkettige Polyole und Polyamine (Kettenverlängerer oder Vernetzer), wie sie aus dem Stand der Technik hinlänglich bekannt sind. Dies können beispielsweise niedermolekulare Diole (z.B. 1,2-Ethandiol, 1,3- bzw. 1,2-Propandiol, 1,4-Butandiol), Triole (z.B. Glycerin, Trimethylolpropan) und Tetraole (z.B. Pentaerythrit) sein, aber auch höhermolekulare Polyhydroxyverbindungen wie Polyetherpolyole, Polyesterpolyole, Polycarbonatpolyole, Polysiloxanpolyole, Polyamine und Polyetherpolyamine sowie Polybutadienpolyole.

Polyetherpolyole sind in an sich bekannter Weise durch Alkoxylierung von geeigneten StarterMolekülen unter Basenkatalyse oder Einsatz von Doppelmetallcyanidverbindungen (DMC-Verbindungen) zugänglich. Geeignete Starter-Moleküle für die Herstellung von Polyetherpolyolen sind beispielsweise einfache, niedermolekulare Polyole, Wasser, organische Polyamine mit mindestens zwei N-H-Bindungen oder beliebige Gemische derartiger Starter-Moleküle. Bevorzugte Starter-Moleküle zur Herstellung von Polyetherpolyolen durch Alkoxylierung, insbesondere nach dem DMC-Verfahren, sind insbesondere einfache Polyole wie Ethylenglykol, Propylenglykol-1,3-und Butandiol-1,4, Hexandiol-1,6, Neopentylglykol, 2-Ethylhexandiol-1,3, Glyzerin, Trimethylolpropan, Pentaerythrit sowie niedermolekulare, Hydroxylgruppen aufweisende Ester derartiger Polyole 5 mit Dicarbonsäuren der nachstehende beispielhafte genannten Art oder niedermolekulare Ethoxylierungs-oder Propoxylierungsprodukte derartiger einfacher Polyole oder beliebige Gemische derartiger modifizierter oder nicht modifizierter Alkohole. Für die Alkoxylierung geeignete Alkylenoxide sind insbesondere Ethylenoxid und/oder Propylenoxid, die in beliebiger Reihenfolge oder auch im Gemisch bei der Alkoxylierung eingesetzt werden können.

Polyesterpolyole können in bekannter Weise durch Polykondensation von niedermolekularer Polycarbonsäurederivaten, wie beispielsweise Bernsteinsäure, Adipinsäure, Korksäure, Azelainsäure, Sebacinsäure, Dodekandisäure, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, Dimerfettsäure, Trimerfettsäure, Phthalsäure, Phthalsäureanhydrid, Isophthalsäure, Terephthalsäure, Zitronensäure oder Trimellithsäure, mit niedermolekularen Polyolen, wie beispielsweise Ethylenglykol, Diethylenglykol, Neopentylglykol, Hexandiol, Butandiol, Propylenglykol, Glycerin, Trimethylolpropan 1,4-Hydroxymethylcyclohexan, 2-Methyl-1,3-propandiol, Butantriol-1,2, 4, Triethylenglykol, Tetraethylenglykol, Polyethylenglykol, Dipropylenglykol, Polypropylenglykol, Dibutylenglykol und Polybutylenglykol, oder durch Ring öffnende Polymerisation cyclischer Carbonsäureester, wie *ε*-Caprolacton, herstellen. Darüber hinaus lassen sich auch Hydroxycarbonsäurederivate, wie beispielsweise Milchsäure, Zimtsäure oder *ω*-Hydroxycapronsäure zu Polyesterpolyolen polykondensieren. Es können aber auch olyesterpolyole oleochemischer Herkunft verwendet werden. Derartige Polyesterpolyole können beispielsweise durch vollständige Ringöffnung von epoxidierten Triglyceriden eines wenigstens teilweise olefinisch ungesättigte Fettsäureenthaltenden Fettgemisches mit einem oder mehreren Alkoholen mit 1 bis 12 C-Atomen und anschließender partieller Umesterung der Triglycerid-Derivate zu Alkylesterpolyolen mit 1 bis 12 C-Atomen im Alkylrest hergestellt werden.

Die NCO-reaktive Verbindung kann als Vernetzerkomponente bzw. Kettenverlängerer kurzkettige Polyole bzw. Polyamine enthalten. Typische Kettenverlängerer sind Diethylentoluoldiamin (DETDA), 4,4'-Methylenbis-(2,6-diethyl)-anilin (MDEA), 4,4'-Methylenbis-(2,6-diisopropyl)-anilin (MDIPA), 4,4'-Methylen-bis-(3-chloro-2,6-diethyl)-anilin (MCDEA), Dimethylthiotoluoldiamin (DMTDA, Ethacure® 300), N,N'-Di(sec-butyl)-amino-biphenylmethan (DBMDA, Unilink® 4200) oder N,N'-Di-sec-butyl-p-phenylendiamin (Unilink® 4100), 3,3'-Dichloro-4,4' -diaminodiphenylmethan (MBOCA), Trimethylenglykol-di-p-aminobenzoat (Polacure 740M). Aliphatische aminische Kettenverlängerer können ebenfalls eingesetzt oder mitverwendet werden. 1,3-Propandiol, 1,4-Butandiol, 2,3-Butandiol, 1,5-Pentandiol, 1,6-Hexandiol und HQEE (Hydrochinondi(*β*-hydroxyethyl)ether) sowie Wasser. Ganz besonders bevorzugt wird 1,4-Butandiol für massive Gießelastomere und Wasser für zellige Gießelastomere verwendet.

Eine Übersicht über Polyurethane, ihre Eigenschaften und Anwendungen wird beispielsweise im Kunststoffhandbuch, Band 7, Polyurethane, 3. neu bearbeitete Auflage, Band 193, herausgegeben von Prof. Dr. G.W. Becker und Prof. Dr. D. Braun (Carl-Hanser-Verlag, München, Wien) gegeben.

Bevorzugt werden NCO-terminierte Prepolymere mit einem NCO-Gehalt von 2 bis 15 Gew.-%, ganz besonders von 2-10 Gew.-% eingesetzt. Das bei Raumtemperatur feste Diisocyanat wird bevorzugt mit Polyolen der Funktionalität 2 bis 3 bevorzugt 2 und einer OH-Zahl von 28-112 mg 10 KOH/g Substanz zu Prepolymeren umgesetzt. Bevorzugt werden Ester basierte Polyole eingesetzt. Die so hergestellten NCO-Prepolymere werden entweder direkt weiter umgesetzt oder als lagerstabile Prepolymere in beispielsweise Fässern bis zu ihrer endgültigen Verwendung gelagert. Bevorzugt werden 1,5-NDI basierte Prepolymere eingesetzt. Die Herstellung der Gießelastomere (Formteile) wird vorteilhaft bei einem NCO/OH Verhältnis von 0,7 bis 1,30 durchgeführt. Im Falle von zelligen Elastomeren wird die Menge des in das Formwerkzeug eingebrachten Gemisches üblicherweise so bemessen, dass die erhaltenen Formkörper die bereits dargestellte Dichte aufweisen. Die Ausgangskomponenten werden üblicherweise mit einer Temperatur von 30 bis 110 °C in das Formwerkzeug eingebracht. Die Verdichtungsgrade liegen zwischen 1,1 und 8, vorzugsweise zwischen 2 und 6. Die zelligen Elastomere werden zweckmäßigerweise mit einer Niederdruck-Technik oder insbesondere der Reaktionsspritztechnik (RIM) in offenen, bevorzugt geschlossenen Formwerkzeugen hergestellt.

Die Reaktionsspritzguß-Technik wird beispielsweise beschrieben von H. Piechota und H. Röhr in "Integral Schaumstoffe", Carl Hanser-Verlag, München, Wien 1975; D.J. Prepelka und J.L. Wharton in Journal of Cellular Plastics, März/April 1975, Seiten 87 bis 98 und U. Knipp 25 in Journal of CellularPlastics, März/April 1973, Seiten 76-84.

Zusatzstoffe wie Rizinusöl oder Carbodiimide (bspw. Stabaxole der Rheinchemie als Hydrolyseschutzmittel, 2,2',6,6'-Tetraisopropyldiphenylcarbodiimid ist ein bekannter Vertreter) können sowohl dem Polyol als auch dem Prepolymer zugesetzt werden. Wasser, Emulgatoren, Katalysatoren und/oder Hilfs- und/oder Zusatzstoffe bilden mit dem Polyol gängigerweise die Polyolkomponente.

Zur besseren Entformung ist es üblich die Formwerkzeuge mit äußeren Trennmitteln zu versehen, beispielsweise Verbindungen auf Wachs- oder Silikonbasis oder wässrige Seifenlösungen. Die entformten Formkörper werden üblicherweise 1 bis 48 Stunden bei Temperaturen von 70 bis 120 °C nachgetempert.

Als Emulgator werden beispielsweise sulfonierte Fettsäuren sowie weitere allgemein bekannte Emulgatoren eingesetzt, wie z. B. Polyglykolester von Fettsäuren, Alkylarylpolyglykolether, Alkoxylate von Fettsäuren, bevorzugt Polyethylenglykolester, Polypropylenglykolester, Polyethylenpolypropylenglykolester, Ethoxylate und/oder Propoxylate der Linolsäure, 5 Linolensäure, Ölsäure, Arachidonsäure, besonders bevorzugt Ölsäureethoxylate. Alternativ können auch Polysiloxane verwendet werden. Salze von Fettsäuren mit Aminen, z.B. ölsaures Diethylamin, stearinsaures Diethanolamin, ricinolsaures Diethanolamin, Salze von Sulfonsäuren, z.B. Alkalioder Ammoniumsalze von Dodecylbenzol- oder Dinaphthylmethandisulfonsäure sind ebenfalls bevorzugt.

Die sulfonierten Fettsäuren können bevorzugt als wässrige Lösungen, beispielsweise als 50%-ige Lösung eingesetzt werden. Typische bekannte Produkte sind Zusatzmittel SV und SM von Rheinchemie, sowie als nicht wässriger Emulgator Zusatzmittel WM von Rheinchemie.

Das Verfahren zur Herstellung der zelligen PUR-Gießelastomere wird in Gegenwart von Wasser durchgeführt. Das Wasser wirkt sowohl als Vernetzer unter Bildung von Harnstoffgruppen als auch 15 aufgrund der Reaktion mit Isocyanatgruppen unter Bildung von Kohlendioxid als Treibmittel. Die Wassermengen, die zweckmäßigerweise verwendet werden können, betragen 0,01 bis 5 Gew.-%, vorzugsweise 0,3 bis 3,0 Gew.-%, bezogen auf das Gewicht der Polyolkomponente. Das Wasser kann vollständig oder teilweise in Form der wässrigen Lösungen der sulfonierten Fettsäuren eingesetzt werden.

Die Katalysatoren können einzeln wie auch in Abmischung miteinander zugegeben werden. Vorzugsweise sind dies metallorganische Verbindungen, wie Zinn-(II)-Salze von organischen Carbonsäuren, z. B. Zinn-(II)-dioctoat, Zinn-(II)-dilaurat, Dibutylzinndiacetat und Dibutylzinndilaurat und tertiäre Amine wie Tetramethyl-ethylendiamin, N-Methylmorpholin, Diethylbenzylamin, Triethylamin, Dimethylcyclohexylamin, Diazabicyclooctan, N,N'-Dimethylpiperazin, N-Methyl-N'-(4-N-Dimethylamino-)butylpiperazin, N,N,N',N",N"-Pentamethyldiethylentriamin oder ähnliche. Weiterhin kommen als Katalysatoren in Betracht: Amidine, wie z.B. 2,3-Dimethyl-3,4,5,6-tetrahydropyrimidin, Tris-(dialkyIamino-alkyl)-shexahydrotriazine, insbesondere Tris-(N,N-dimethylamino-propyl)-s-hexahydrotriazin, Tetraalkylammoniumhydroxide, wie z.B. Tetramethylammoniumhydroxid, Alkalihydroxide, wie 30 z.B. Natriumhydroxid, und Alkalialkoholate, wie z.B. Natriummethylat und Kaliumisopropylat, sowie Alkalisalze von langkettigen Fettsäuren mit 10 bis 20 C-Atomen und gegebenenfalls seitenständigen OH Gruppen. Je nach einzustellender Reaktivität gelangen die Katalysatoren in Mengen von 0,001 bis 0,5 Gew.-%, bezogen auf die Isocyanatkomponente zur Anwendung.

Die erfindungsgemäßen Elastomere, insbesondere Polyurethane oder Formkörper unterscheiden sich von den im Stand der Technik bekannten auf monomeren bei Raumtemperatur festen Diisocyanaten, bevorzugt auf 1,5-Naphthalindiisocyanat basierenden Produkten dadurch, dass durch das erfindungsgemäße Verfahren auch die Kontamination des aus dem Rückstand zurückgewonnenen, monomeren bei Raumtemperatur festen Diisocyanats mit diesen Hilfsstoffen minimiert.

Verwendung finden derartige zellige PUR-Gießelastomere, auch als Formkörper bezeichnet, als Dämpfungselemente im Fahrzeugbau, beispielsweise im Automobilbau, z. B. als Zusatzfedern, Anschlagpuffer, Querlenkerlager, Hinterachsenfahrschemellager, Stabilisator-Lager, Längsstreben-Lager, Federbein-Stützlager, Stoßdämpferlager, Lager für Dreieckslenker und als auf der Felge befindliches Notrad, das beispielsweise bei einem Reifenschaden bewirkt, dass das Fahrzeug auf dem zelligen Elastomer fährt und steuerbar bleibt. Die massiven Gießelastomere können auch als Beschichtung für Rollen, Räder und Walzen, Rakel, Siebe oder Hydrozyklone verwendet werden. Die Erfindung betrifft in einer ersten Ausführungsform ein Verfahren zur Abtrennung eines bei Raumtemperatur festen Diisocyanats aus einem Destillationsrückstand, aus einem Herstellungsprozess des Diisocyanats, umfassend die folgenden Schritte:
(i) Vermischen des Destillationsrückstandes mit einem Bitumen in der Weise, dass eine Mischung erhalten wird, welche 70 bis 90 Gew.-% des Destillationsrückstandes und 10 bis 30 Gew.-% des Bitumens enthält, jeweils bezogen auf die Mischung,
(ii) Destillation der Mischung in einem Dünnschichtverdampfer oder einem Fallfilmverdampfer, wobei ein Sumpfablauf und ein gasförmiger Produktstrom erhalten wird,
(iii) Kondensation des gasförmigen Produktstroms und Erhalt eines Feststoffs enthaltend das bei Raumtemperatur feste Diisocyanat.

In einer zweiten Ausführungsform betrifft die Erfindung ein Verfahren nach Ausführungsform 1, dadurch gekennzeichnet, dass das Bitumen der Mischung aus Schritt (i) einen Schwefelgehalt von 2,5 bis 3,1 Gew.-% aufweist, insbesondere 2,7 bis 3,1 Gew.-%, bevorzugt von 2,9 bis 3,08 Gew.-%, jeweils bezogen auf das Bitumen.

In einer dritten Ausführungsform betrifft die Erfindung ein Verfahren nach Ausführungsform 1 oder 2, dadurch gekennzeichnet, dass der Destillationsrückstand in Schritt (i) von 30 bis 70 Gew.-% des bei Raumtemperatur festen Diisocyanats enthält, bevorzugt 40 bis 60 Gew.-%, besonders bevorzugt 45 bis 50 Gew.-%, jeweils bezogen auf den Destillationsrückstand.

In einer vierten Ausführungsform betrifft die Erfindung ein Verfahren nach einer der Ausführungsformen 1 bis 3, dadurch gekennzeichnet, dass in Schritt (i) 85 bis 70 Gew.-% des Destillationsrückstands mit von 15 bis 30 Gew.-% des Bitumens vermischt werden, jeweils bezogen auf die Summe der Massen des Destillationsrückstands und des Bitumens.

In einer fünften Ausführungsform betrifft die Erfindung ein Verfahren nach einer der Ausführungsformen 1 bis 4, dadurch gekennzeichnet, dass Schritt (ii) bei einer Temperatur von 130 °C bis 160 °C und einem Druck von 0,4 bis 4 mbar durchgeführt wird, bevorzugt von 140°C bis 160 °C und von 0,6 mbar bis 2 mbar, besonders bevorzugt von 150 °C bis 160 °C und von 0,7 bis 1,5 mbar.

In einer sechsten Ausführungsform betrifft die Erfindung ein Verfahren nach einer der Ausführungsformen 1 bis 5, dadurch gekennzeichnet, dass der Sumpfablauf aus Schritt (ii) bei der vorherrschenden Temperatur am Ausgang des Dünnschichtverdampfers oder Fallfilmverdampfers kein Feststoff ist.

In einer siebten Ausführungsform betrifft die Erfindung ein Verfahren nach einer der Ausführungsformen, 1 bis 6, dadurch gekennzeichnet, dass der Feststoff aus Schritt (iii) wenigstens 95 Gew.-% des bei Raumtemperatur festen Diisocyanats enthält, bevorzugt wenigstens 97 Gew.-%, besonders bevorzugt wenigstens 99 Gew.-%, jeweils bezogen auf den Feststoff.

In einer achten Ausführungsform betrifft die Erfindung ein Verfahren nach einer der Ausführungsformen 1 bis 7, dadurch gekennzeichnet, dass der Herstellungsprozess des Diisocyanats eine Phosgenierung eines Diamins ist, bevorzugt eine Flüssigphasen-Phosgenierung eines Diamins.

In einer neunten Ausführungsform betrifft die Erfindung ein Verfahren nach einer der Ausführungsformen 1 bis 8, dadurch gekennzeichnet, dass das bei Raumtemperatur feste Diisocyanat 1,5-Naphthalindiisocyanat, 1,8-Naphthalindiisocyanat, 1,4-Phenylendiisocyanat, Tetralindiisocyanat, o-Tolidindiisocyanat, Duroldiisocyanat, Benzidindiisocyanat und/oder 1,4-Anthrylendiisocyanat, bevorzugt 1,5-Naphthalindiisocyanat, 1,8-Naphthalindiisocyanat, 1,4-Phenylendiisocyanat, Tetralindiisocyanat und/oder o-Tolidindiisocyanat und besonders bevorzugt 1,5-Naphthalindiisocyanat und/oder 1,8-Naphthalindiisocyanat ist.

In einer zehnten Ausführungsform betrifft die Erfindung die Verwendung einer Mischung enthaltend 70 bis 90 Gew.-% eines Destillationsrückstandes aus einem Herstellungsprozess eines bei Raumtemperatur festen Diisocyanats und 10 bis 30 Gew.-% eines Bitumens, jeweils bezogen auf die Mischung, in einem Verfahren zur Abtrennung des Diisocyanats durch Destillation mittels eines Dünnschichtverdampfers oder Fallfilmverdampfers.

In einer elften Ausführungsform betrifft die Erfindung eine Zusammensetzung umfassend einen Feststoff enthaltend ein bei Raumtemperatur festes Diisocyanat aus Schritt (iii) eines Verfahrens nach einer der Ausführungsformen 1 bis 9 und mindestens eine NCO-reaktive Verbindung, bevorzugt mindestens ein Polyesterpolyol.

In einer zwölften Ausführungsform betrifft die Erfindung ein Verfahren zur Herstellung eines Elastomers, bei dem mindestens eine Zusammensetzung gemäß Ausführungsform 11, gegebenenfalls unter Erwärmen, chemisch umgesetzt wird.

In einer zwölften Ausführungsform betrifft die Erfindung ein Elastomer, hergestellt oder herstellbar nach einem Verfahren gemäß Ausführungsform 12.

Die vorliegende Erfindung wird im Folgenden anhand von Beispielen und Vergleichsbeispielen näher erläutert, ohne sie jedoch auf diese einzuschränken.

### Beispiele

Monomeres Naphtalindiisocyanat (NDI) wurde aus Destillationsrückständen mit verschiedenen Verfahren abgetrennt. Dazu wurde der Destillationsrückstand mit verschiedenen Bitumenarten gemischt und einer Destillation unterworfen. Es wurde folgendes Bitumen verwendet
Bitumen 70/100 von Shell: 3,5 Gew.-% Schwefel
Bitumen 160/220 von Shell: 2,9 Gew.-% Schwefel
Bitumen mit 3,08 Gew.-% Schwefel, Mischung aus Bitumen 70/100 und 160/220
Bitumen mit 3,23 Gew.-% Schwefel Mischung aus Bitumen 70/100 und 160/220

Die Bestimmung des Schwefelgehaltes erfolgte durch Schwefel-Elementaranalyse nach Rohrverbrennung im Automaten bei 1150°C unter Sauerstoffzugabe. Anschließend erfolgt die Detektion mit einem IR-Detektor als SO₂.

Die Reinheit des NDIs wurde gaschromatographisch bestimmt. Die Messungen erfolgten auf einem HP 6890 von Hewlett Packard mit FID-Detektor und der HP-Chemstation Software unter 5 Verwendung einer Optima 5 Säule und folgenden Parametern: Split-Rate: 8.31:1 mL/min; Flussrate: 96,4 mL/min Druck: 0,7 bar, Trägergas: Helium, Injektionsvolumen: 1 *µ*L, Inliner: gerader Split-Liner gefüllt mit Carbofritt.

Die NDI-Rückstände vor und nach der Destillation wurden mittels GPC nach DIN 55672-1:2007-08 analysiert. Die Ausbeutebestimmung erfolgte dann durch Subtraktion der Flächenprozent des noch verbleibenden Monomers von den Flächenprozent der ursprünglichen Monomermenge, welche jeweils durch GPC nach der DIN 55672-1:2007-08 bestimmt wurden.

| **Beispiel** | **Erfindungsgemäß** | | | | | | | **Vergleich** |
|---|---|---|---|---|---|---|---|---|
| **Gemisch** | **1** | **2** | **3** | **4** | **5** | **6** | **7** | **8** |
| Destillationsrückstand aus der Phosghenierung von 1,5-NDA zu 1,5-NDI [Gew.-%] | 70 | 70 | 85 | 70 | 70 | 70 | 90 | 60 |
| Bitumen 160/220 (2,9 Gew.-% Schwefel) [Gew.-%] | 30 | 30 | 15 | - | - | - | 10 | |
| Bitumen mit 3,08 Gew.-% Schwefel [Gew.-%] | - | - | - | 30 | - | - | - | |
| Bitumen mit 3,32 Gew.-% Schwefel [Gew.-%] | - | - | - | - | - | 30 | - | |
| Bitumen 70/100 (3,5 Gew.-% Schwefel) [Gew.-%] | - | - | - | - | 30 | - | - | 40 |
| Anteil an momoren 1,5 NDI im Destillationsrückstand [Gew.-% bezogen auf den Destillationsrückstand] | 46,9 | 46,9 | 46,9 | 46,9 | 46,9 | 46,9 | 46,9 | 45,9 |

| **Destillationsbedingungen** | | | | | | | | |
|---|---|---|---|---|---|---|---|---|
| Temperatur [°C] | 155 | 155 | 155 | 155 | 155 | 155 | 155 | 160 |
| Druck [mbar] | 0,9 | 0,9 | 0,9 | 0,9 | 0,9 | 0,9 | 0,9 | 0,9 |
| Dosierrate [g/h] | 230 | 265 | 265 | 265 | 265 | 265 | 265 | 260 |
| **Anteil ein zurückgewonnenem momoren 1,5 NDI Monomer (Ausbeute)** [%] | 84,5 | 86,4 | 87,1 | 87,3 | 62,8 | 65,8 | 79,3 | 78,7 |
| Anteil an 1,5-NDI-Monomer im Sumpfablauf [%] | 7,3 | 8,3 | 6,1 | 6,0 | 17,4 | 17,4 | 9,7 | 9,8 |
| Reinheit 1,5-NDI-Monomer [%] | 99,5 | 99,9 | - | - | - | - | - | 99,7 |

Die Angaben in Gew.-% für die Mischung beziehen sich auf die Masse die gesamte Mischung. Die jeweilige Mischung wurde unter den angegeben Bedinungen einer Vakuumdestillation in einem Dünnschichtverdampfer zugeführt, wobei das monomere 1,5-NDI als Feststoff kondensiert wurde. Der bei dieser Temperatur noch flüssige Sumpfablauf bestand jeweils aus 1,5-NDI-Monomer, nicht-destillierbaren Anteilen und Bitumen. Alle Beispiele und Vergleichsbeispiele (ausgenommen Vergleichsbeispiel 9) wurden auf einem handelsüblichen Fallfilmverdampfer aus Glas, mit einer Verdampferfläche von 0.1 m² (Durchmesser 100 mm, Länge 300 mm) durchgeführt.

### Vergleichsbeispiel 9 (ohne Dünnschichtverdampfer)

Rückstand aus der Phosgenierung von 1,5-NDA zu 1,5-NDI, noch enthaltend 70-85% 1,5-NDI-Monomer, wurde bei 150 °C einer auf 160 °C vorgeheizten Menge Bitumen (30 Gew.-% des Rückstandes) in einem Kessel zugeführt. Aus dem erhaltenen Gemisch wurde unter Rühren kontinuierlich bei 2-4 mbar und 160 °C 1,5-NDI-Monomer abdestilliert. Die Ausbeute des zurückgewonnen 1,5-NDI-Monomers betrug bei diesem Verfahren 50-60%.

### Diskussion der Ergebnisse

Der Vergleich der erfindungsgemäßen Beispiele mit den Vergleichsbeispielen zeigt, dass mit dem erfindungsgemäßen Verfahren ein vergleichbarer oder sogar ein ein höherer Anteil von mehr als 80 % des monomeren Diisocyanats aus dem Destillationsrückstand zurückgewonnen werden kann. In den Vergleichsbeispiel 8 wurde zwar auch eine Ausbeute von fast 80% erzielt, allerdings nur indem der Destillationsrückstand mit 40 Gew.-% Bitumen vermischt wurde, also mehr Zusatzsstoffe verbraucht wurden.

Ein weiterer Vorteil des erfindungsgemäßen Verfahrens ist, dass der Sumpfablauf fließfähig ist, da weitere Oligomerisierungsreaktionen durch die im Vergleich mit herkömmlichen Verfahren niedrigen Temperaturen bei der Durchführung des erfindungsgemäßen Verfahrens weitestgehend unterdrückt werden, was einen bedeutenden Vorteil für die kontinuierliche Fahrweise darstellt. Die aus dem erfindungsgemäßen Verfahren erhaltenen bei Raumtemperatur festen Diisocyanate zeichnen sich durch eine hohe Reinheit aus und können ohne Einschränkungen zur Herstellung von Elastomeren eingesetzt werden.

## Patentansprüche

1. Verfahren zur Abtrennung eines bei Raumtemperatur festen Diisocyanats aus einem Destillationsrückstand, aus einem Herstellungsprozess des Diisocyanats, umfassend die folgenden Schritte:
(i) Vermischen des Destillationsrückstandes mit einem Bitumen in der Weise, dass eine Mischung erhalten wird, welche 70 bis 90 Gew.-% des Destillationsrückstandes und 10 bis 30 Gew.-% des Bitumens enthält, jeweils bezogen auf die Mischung,
(ii) Destillation der Mischung in einem Dünnschichtverdampfer oder einem Fallfilmverdampfer, wobei ein Sumpfablauf und ein gasförmiger Produktstrom erhalten wird,
(iii) Kondensation des gasförmigen Produktstroms und Erhalt eines Feststoffs enthaltend das bei Raumtemperatur feste Diisocyanat.

2. Verfahren nach Anspruch 1, **dadurch gekennzeichnet, dass** das Bitumen der Mischung aus Schritt (i) einen Schwefelgehalt von 2,5 bis 3,1 Gew.-% aufweist, insbesondere 2,7 bis 3,1 Gew.-%, bevorzugt von 2,9 bis 3,08 Gew.-%, jeweils bezogen auf das Bitumen.

3. Verfahren nach Anspruch 1 oder 2, **dadurch gekennzeichnet, dass** der Destillationsrückstand in Schritt (i) von 30 bis 70 Gew.-% des bei Raumtemperatur festen Diisocyanats enthält, bevorzugt 40 bis 60 Gew.-%, besonders bevorzugt 45 bis 50 Gew.-%, jeweils bezogen auf den Destillationsrückstand.

4. Verfahren nach einem der vorstehenden Ansprüche, **dadurch gekennzeichnet, dass** in Schritt (i) 85 bis 70 Gew.-% des Destillationsrückstands mit von 15 bis 30 Gew.-% des Bitumens vermischt werden, jeweils bezogen auf die Summe der Massen des Destillationsrückstands und des Bitumens.

5. Verfahren nach einem der vorstehenden Ansprüche, **dadurch gekennzeichnet, dass** Schritt (ii) bei einer Temperatur von 130 °C bis 160 °C und einem Druck von 0,4 bis 4 mbar durchgeführt wird, bevorzugt von 140°C bis 160 °C und von 0,6 mbar bis 2 mbar, besonders bevorzugt von 150 °C bis 160 °C und von 0,7 bis 1,5 mbar.

6. Verfahren nach einem der vorstehenden Ansprüche, **dadurch gekennzeichnet, dass** der Sumpfablauf aus Schritt (ii) bei der vorherrschenden Temperatur am Ausgang des Dünnschichtverdampfers oder Fallfilmverdampfers kein Feststoff ist.

7. Verfahren nach einem der vorstehenden Ansprüche, **dadurch gekennzeichnet, dass** der Feststoff aus Schritt (iii) wenigstens 95 Gew.-% des bei Raumtemperatur festen Diisocyanats enthält, bevorzugt wenigstens 97 Gew.-%, besonders bevorzugt wenigstens 99 Gew.-%, jeweils bezogen auf den Feststoff.

8. Verfahren nach einem der vorstehenden Ansprüche, **dadurch gekennzeichnet, dass** der Herstellungsprozess des Diisocyanats eine Phosgenierung eines Diamins ist, bevorzugt eine Flüssigphasen-Phosgenierung eines Diamins.

9. Verfahren nach einem der vorstehenden Ansprüche, **dadurch gekennzeichnet, dass** das bei Raumtemperatur feste Diisocyanat 1,5-Naphthalindiisocyanat, 1,8-Naphthalindiisocyanat, 1,4-Phenylendiisocyanat, Tetralindiisocyanat, o-Tolidindiisocyanat, Duroldiisocyanat, Benzidindiisocyanat und/oder 1,4-Anthrylendiisocyanat, bevorzugt 1,5-Naphthalindiisocyanat, 1,8-Naphthalindiisocyanat, 1,4-Phenylendiisocyanat, Tetralindiisocyanat und/oder o-Tolidindiisocyanat und besonders bevorzugt 1,5-Naphthalindiisocyanat und/oder 1,8-Naphthalindiisocyanat ist.

10. Verwendung einer Mischung enthaltend 70 bis 90 Gew.-% eines Destillationsrückstandes aus einem Herstellungsprozess eines bei Raumtemperatur festen Diisocyanats und 10 bis 30 Gew.-% eines Bitumens, jeweils bezogen auf die Mischung, in einem Verfahren zur Abtrennung des Diisocyanats durch Destillation mittels eines Dünnschichtverdampfers oder Fallfilmverdampfers.

11. Zusammensetzung umfassend einen Feststoff enthaltend ein bei Raumtemperatur festes Diisocyanat aus Schritt (iii) eines Verfahrens nach einem der Ansprüche 1 bis 9 und mindestens eine NCO-reaktive Verbindung, bevorzugt mindestens ein Polyesterpolyol.

12. Verfahren zur Herstellung eines Elastomers, bei dem mindestens eine Zusammensetzung gemäß Anspruch 11, gegebenenfalls unter Erwärmen, chemisch umgesetzt wird.

13. Elastomer, hergestellt oder herstellbar nach einem Verfahren gemäß Anspruch 12.
